(19) Europäisches Patentamt
European Patent Office
Office européen des brevets

(11) EP 0 611 744 B1

(12) EUROPÄISCHE PATENTSCHRIFT

(45) Veröffentlichungstag und Bekanntmachung des Hinweises auf die Patenterteilung:
**13.11.1996 Patentblatt 1996/46**

(51) Int. Cl.$^6$: **C07C 17/00**, C07C 19/08

(21) Anmeldenummer: **94101811.1**

(22) Anmeldetag: **07.02.1994**

# (54) Verfahren zur Herstellung von 1,1,1,3,3-Pentafluorpropan

Process for the preparation of 1,1,1,3,3-pentafluoropropane

Procédé pour la préparation de 1,1,1,3,3-pentfluoropropane

(84) Benannte Vertragsstaaten:
**BE DE ES FR GB IT**

(30) Priorität: **19.02.1993 DE 4305164**

(43) Veröffentlichungstag der Anmeldung:
**24.08.1994 Patentblatt 1994/34**

(73) Patentinhaber: **BAYER AG**
**51368 Leverkusen (DE)**

(72) Erfinder:
- **Bielefeldt, Dietmar, Dr.**
  **D-40883 Ratingen (DE)**
- **Lui, Norbert, Dr.**
  **D-51061 Köln (DE)**
- **Marhold, Albrecht, Dr.**
  **D-51373 Leverkusen (DE)**

(56) Entgegenhaltungen:
**EP-A- 0 442 075**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 611 744 B1

Printed by Rank Xerox (UK) Business Services
2.13.8/3.4

## Beschreibung

Die vorliegende Erfindung betrifft ein besonders vorteilhaftes Verfahren zur Herstellung von 1,1,1,3,3-Pentafluorpropan. 1,1,1,3,3-Pentafluorpropan ist neuerdings von besonderem Interesse, da es als Ersatzstoff für FCKW's eingesetzt werden kann.

Aus J. Am. Chem. Soc. 70, 2023 (1948) ist bekannt, daß es sich bei 1,1,1,3,3-Pentafluor-2,3-dichlorpropan um eine empfindliche Verbindung handelt, die leicht, z.B. in verdünnter wäßriger Alkalilauge, in die ungesättigte Verbindung 1,1,1,3,3-Pentafluor-2-chlorpropen übergeht (siehe Reaktionsgleichung (1)).

$$CF_3\text{-}CHCl\text{-}CF_2Cl \xrightarrow[H_2O/Alkal.]{} CF_3\text{-}CCl{=}CF_2 \quad (1)$$

Aus EP-A 434 408 ist bekannt, daß 2-Chlor-heptafluorpropan durch katalytische Umsetzung mit Wasserstoff in die ungesättigte Verbindung Hexafluorpropen übergeht (siehe Reaktionsgleichung (2)).

$$CF_3\text{-}CFCl\text{-}CF_3 \xrightarrow[H_2/Kat.]{} CF_3\text{-}CF{=}CF_2 \quad (2)$$

Aus DE-A 4 004 495 ist bekannt, daß 1,1,1,3,3,3-Hexafluor-2-chlorpropan durch katalytische Umsetzung mit Wasserstoff bei hohem Umsatz und guter Selektivität in 1,1,1,3,3,3-Hexafluorpropan übergeht (siehe Reaktionsgleichung (3)).

$$CF_3\text{-}CHCl\text{-}CF_3 \xrightarrow[H_2/Kat.]{} CF_3\text{-}CH_2\text{-}CF_3 \quad (3)$$

Gemäß WO 90/8753 werden u.a. $CF_2Cl\text{-}CF_2$-Gruppen enthaltende fluorierte und chlorierte Propane katalytisch mit Wasserstoff umgesetzt. Dabei findet in fast allen Fällen gar keine oder nur in äußerst geringem Umfang eine Hydrodechlorierung der $CF_2Cl$-Gruppe statt. Charakteristische Beispiele für gemäß WO 90/8753 an fluorierten und chlorierten Propanen mit $CF_2Cl$-Gruppen ablaufende Reaktionen sind in der Tabelle 1 zusammengestellt.

Tabelle 1

| Beispiel Nr. aus WO 90/8753 | eingesetzte Verbindung | Selektivität der Hydrodehalogenierung an einzelnen Atomgruppen | | |
|---|---|---|---|---|
| 4-11 | $CFCl_2$-$CF_2$-$CF_2Cl$ | $CFCl_2$ | i | 99,6 % |
| | | $CF_2Cl$ | i | 3,9 % |
| | | $CF_2Cl$ | e | 0,1 % |
| 4-25 | $CFH_2$-$CF_2$-$CF_2Cl$ | $CFH_2$ | i | 98,1 % |
| | | $CF_2Cl$ | i | 11,2 % |
| | | $CF_2Cl$ | e | 1,1 % |
| 4-33 | $CFCl_2$-$CF_2$-$CF_2Cl$ | $CFCl_2$ | i | (98,8 %)*) |
| | | $CF_2Cl$ | i | (98,8 %)*) |
| | | $CF_2Cl$ | e | - |
| 6-11 | $CCl_2H$-$CF_2$-$CF_2Cl$ | $CCl_2H$ | i | 98,8 % |
| | | $CF_2Cl$ | i | 0,3 % |
| | | $CF_2Cl$ | e | - |
| 6-21 | $CCl_3$-$CF_2$-$CF_2Cl$ | $CCl_3$ | i | 95,7 % |
| | | $CF_2Cl$ | i | - |
| | | $CF_2Cl$ | e | - |
| 6-26 | $CClH_2$-$CF_2$-$CF_2Cl$ | $CClH_2$ | i | 98,1 % |
| | | $CF_2Cl$ | i | 1,9 % |
| | | $CF_2Cl$ | e | - |
| 6-30 | $CH_3$-$CF_2$-$CF_2Cl$ | $CH_3$ i | | (68,9 %)*) |
| | | $CF_2Cl$ | i | (68,9 %)*) |
| | | $CF_2Cl$ | e | - |

In Tabelle 1 bedeutet "e" die Selektivität für die Hydrodechlorierung, die selektiv an der $CF_2Cl$-Gruppe abläuft, d.h. ohne daß Reaktionen an anderen Atomgruppen stattfinden. "i" bedeutet die Selektivität für Hydrodehalogenierungen an der jeweils angegebenen Atomgruppe, unabhängig davon, ob auch Reaktionen an anderen Atomgruppen stattfinden (inklusive). Die mit *) bezeichneten Reaktionen sind keine Hydrodehalogenierungen, sondern Isomerisierungen.

Aus alledem ergibt sich, daß bei fluorierten und chlorierten Propanen, die einen $CF_2Cl$-CClX-Molekülteil enthalten (X = H oder Cl), dieser Molekülteil offenbar nicht mit akzeptablen Selektivitäten in einen $CF_2H$-$CH_2$-Molekülteil überführen läßt, ohne das ganz überwiegend andere Reaktionen ablaufen.

Zur Herstellung von $CF_3$-CClX-$CF_2H$ ist bisher nur ein Verfahren bekannt (US 3 585 245), bei dem in einer mehrstufigen, umständlichen Synthese von $CF_3$-CO-$CF_2Cl$ ausgegangen wird und nur unzureichende Ausbeuten erzielbar sind.

Es wurde nun ein Verfahren zur Herstellung von 1,1,1,3,3-Pentafluorpropan gefunden, das dadurch gekennzeichnet ist, daß man fluorierte und chlorierte Propanderivate der Formel (I)

$$CF_3\text{-}CClX\text{-}CF_2Cl \qquad (I)$$

in der

X für Wasserstoff oder Chlor steht,

katalytisch hydriert.

Das erfindungsgemäße Verfahren kann mit folgender Reaktionsgleichung (4) illustriert werden:

$$CF_3 - CClX - CF_2Cl \xrightarrow[H_2/Kat.]{} CF_3 - CH_2 - CF_2H \qquad (4)$$
$$(X=H,Cl)$$

In das erfindungsgemäße Verfahren kann man 1,1,1,3,3-Pentafluor-2,3-dichlorpropan oder 1,1,1,3,3-Pentafluor-2,2,3-trichlorpropan oder beliebige Mischungen beider einsetzen. Bevorzugt kommt 1,1,1,3,3-Pentafluor-2,3-dichlorpropan ($CF_3CClHCF_2Cl$) zum Einsatz.

Einsatzprodukte für das erfindungsgemäße Verfahren sind beispielsweise ausgehend von Pentachlorpropen entweder durch katalytische Umsetzung mit Fluorwasserstoff oder durch gleichzeitige Umsetzung mit Fluorwasserstoff und Chlor zugänglich. Man kann auch 1,1,1-Trifluor-2,3,3-trichlorpropen-2 mit Fluorwasserstoff und Chlor umsetzen und so 1,1,1,3,3-Pentafluor-2,2,3-trichlorpropan erhalten.

Als Wasserstoff für die erfindungsgemäße katalytische Hydrierung kann man handelsüblichen Wasserstoff einsetzen, der frei von Schwefelwasserstoff ist und beispielsweise eine Reinheit von 99,9 % oder mehr hat.

Als Katalysatoren kommen übliche Hydrierkatalysatoren in Frage, insbesondere solche, die Nebengruppenelemente in elementarer Form oder in Form von Verbindungen, gegebenenfalls auf einem Trägermaterial aufgebracht, enthalten. Geeignete Nebengruppenelemente sind insbesondere Nickel, Palladium und Platin. Besonders bevorzugte Katalysatoren enthalten elementares Palladium auf einem Träger, beispielsweise auf Aktivkohle, Spinellen oder anderen für solche Zwecke bekannten Trägermaterialien.

Es ist vorteilhaft, den Wasserstoff im Überschuß einzusetzen. Beispielsweise kann man pro Mol Edukt 1,8 bis 50 Mol, vorzugsweise 2 bis 20 Mol Wasserstoff einsetzen.

Bei der erfindungsgemäßen katalytischen Hydrierung kann das Edukt in der Gasphase oder in der flüssigen Phase vorliegen. Vorzugsweise arbeitet man mit gasförmigem Edukt an einem festangeordneten Katalysator.

Das erfindungsgemäße Verfahren kann beispielsweise bei Temperaturen von 50 bis 500°C und Drucken von 0,1 bis 50 bar durchgeführt werden. Bevorzugt sind Temperaturen von 150 bis 450°C und Drucke von 0,1 bis 5 bar.

Im allgemeinen ist es vorteilhaft, die Katalysatoren, insbesondere Trägerkatalysatoren, die für kontinuierliche Verfahren in der Gasphase vorgesehen sind, vor der Beaufschlagung mit Edukt zu konditionieren. Das kann z.B. durch Überleiten von Stickstoff und anschließend von Wasserstoff erfolgen, jeweils bei erhöhter Temperatur.

Das erfindungsgemäße Verfahren kann kontinuierlich oder diskontinuierlich durchgeführt werden, vorzugsweise arbeitet man kontinuierlich.

Bei der erfindungsgemäßen katalytischen Hydrierung entsteht als Nebenprodukt Chlorwasserstoff. Dieser kann aus dem Reaktionsgemisch beispielsweise durch Waschen mit Wasser entfernt werden.

Bei einer beispielhaft erläuterten kontinuierlichen Ausführungsform des erfindungsgemäßen Verfahrens werden die den Reaktor verlassenden Produkte zunächst mit Wasser gewaschen und dann durch Kondensation gesammelt. In vielen Fällen kann dieses Kondensat direkt weiter verwendet werden. Erwünschtenfalls kann man daraus, z.B. durch Destillation, gereinigtes 1,1,1,3,3-Pentafluorpropan erhalten.

Im Hinblick auf den eingangs geschilderten Stand der Technik ist ausgesprochen überraschend, daß mit dem erfindungsgemäßen Verfahren 1,1,1,3,3-Pentafluorpropan bei Umsätzen von über 99 % in Selektivitäten von über 90 % hergestellt werden kann.

Beispiele

Herstellung von geeigneten Ausgangsprodukten (nicht erfindungsgemäß)

A)

In einem Laborautoklaven wurden 800 ml Fluorwasserstoff und 60 g Antimonpentachlorid vorgelegt und 500 g Hexachlorpropen bei Raumtemperatur zugetropft. Anschließend wurde Stickstoff aufgedrückt (10 bar) und die Reaktionsmischung 5 Stunden lang auf 130°C erhitzt, wobei der entstehende Chlorwasserstoff kontinuierlich entspannt wurde. Nach Abkühlen auf Raumtemperatur wurde die Reaktionsmischung auf Eis gegossen und die organische Phase abgetrennt. So wurden 325 g 1,1,1,3,3-Pentafluor-2,3-dichlorpropan mit einer Reinheit von 96 Gew.-% erhalten.

B)

In einem Laborautoklaven wurden 600 ml Fluorwasserstoff und 45 g Antimonpentachlorid vorgelegt und 270 g 1,1,1-Trifluor-2,3,3-trichlorpropen-2 bei Raumtemperatur zugetropft. Anschließend wurde Stickstoff aufgedrückt (10

bar) und die Reaktionsmischung 3 Stunden lang auf 120°C erhitzt, wobei der entstehende Chlorwasserstoff kontinuierlich entspannt wurde. Nach dem Abkühlen auf Raumtemperatur wurde die Reaktionsmischung auf Eis gegossen und die organische Phase abgetrennt. So wurden 212 g 1,1,1,3,3-Pentafluor-2,3-dichlorpropan in einer Reinheit von 98 Gew.-% erhalten.

Erfindungsgemäße Beispiele

Allgemeines:

Die folgenden Beispiele wurden in einem senkrecht angeordneten, elektrisch beheizten Reaktionsrohr aus Nickel (Länge 31 cm, Durchmesser 3,6 cm), in das 150 ml Trägerkatalysatoren eingefüllt worden waren, durchgeführt. Vor Beginn der Reaktion wurde der Katalysator 1 Stunde lang mit 25 Nl Stickstoff pro Stunde und 1,5 Stunden mit 45 Nl Wasserstoff pro Stunde konditioniert, wobei die Temperatur jeweils bei 225°C lag.

Danach wurden die folgenden Hydrierungen durchgeführt. Dabei wurden die den Reaktor verlassenden Produkte zuerst mit Wasser von Salzsäuregas gereinigt und dann bei -78°C kondensiert. Die Analyse der Zusammensetzung der Reaktionsprodukte erfolgte mit Hilfe einer gekoppelten Gaschromatographie/Massenspektroskopie und mit $^{1}$H- und $^{19}$F-NMR- Spektroskopie. Der Umsatz lag bei allen Beispielen über 99 %.

Im Einzelnen wurden folgende Hydrierungen durchgeführt:

Beispiel 1:

| | |
|---|---|
| Einsatzmengen: | 0,2 Mol/h $CF_3$-CHCl-$CF_2$Cl und<br>0,54 Mol/h Wasserstoff |
| Reaktionsbedingungen: | 250°C, Normaldruck |
| Katalysator: | Palladium auf Aktivkohle (5 g/l) |
| Katalysatorbelastung: | 273 g/l • h |

$CF_3$-$CH_2$-$CF_2$H wurde in einer Selektivität von 93,5 % erhalten.

Beispiel 2

| | |
|---|---|
| Einsatzmengen: | 0,2 mol/h $CF_3$-CHCl-$CF_2$Cl und<br>0,54 mol/h Wasserstoff |
| Reaktionsbedingungen: | 300°C, Normaldruck |
| Katalysator: | Palladium auf Aktivkohle (5 g/l) |
| Katalysatorbelastung: | 271 g/l • h |

$CF_3$-$CH_2$-$CF_2$H wurde in einer Selektivität von 95 % erhalten.

Beispiel 3

| | |
|---|---|
| Einsatzmengen: | 0,2 mol/h $CF_3$-CHCl-$CF_2$Cl und<br>0,8 mol/h Wasserstoff |
| Reaktionsbedingungen: | 350°C, Normaldruck |
| Katalysator: | Palladium auf Aktivkohle (5 g/l) |
| Katalysatorbelastung: | 271 g/l • h |

$CF_3$-$CH_2$-$CF_2$H wurde in einer Selektivität von 91,5 % erhalten.

Beispiel 4

| | |
|---|---|
| Einsatzmengen: | 0,1 mol/h $CF_3$-CHCl-$CF_2$Cl und<br>1,35 mol/h Wasserstoff |
| Reaktionsbedingungen: | 300°C, Normaldruck |
| Katalysator: | Palladium auf MgO/$Li_2Al_2O_4$ (10 g/l) |
| Katalysatorbelastung: | 140 g/l • h |

$CF_3$-$CH_2$-$CF_2$H wurde in einer Selektivität von 91,3 % erhalten.

Beispiel 5

| | |
|---|---|
| Einsatzmengen: | 0,22 mol/h $CF_3$-$CCl_2$-$CF_2Cl$ und<br>1,61 mol/h Wasserstoff |
| Reaktionsbedingungen: | 300°C, Normaldruck |
| Katalysator: | Palladium auf Aktivkohle (5 g/l) |
| Katalysatorbelastung: | 223 g/l • h |

$CF_3$-$CH_2$-$CF_2H$ wurde in einer Selektivität von 93,5 % erhalten.

## Patentansprüche

1. Verfahren zur Herstellung von 1,1,1,3,3-Pentafluorpropan, dadurch gekennzeichnet, daß man fluorierte und chlorierte Propanderivate der Formel (I)

$$CF_3\text{-}CClX\text{-}CF_2Cl \quad (I)$$

in der

X für Wasserstoff oder Chlor steht,

katalytisch hydriert.

2. Verfahren nach Anspruch 1, dadurch gekennzeichnet, daß man 1,1,1,3,3-Pentafluor-2,3-dichlorpropan oder 1,1,1,3,3-Pentafluor-2,2,3-trichlorpropan oder beliebige Mischungen beider einsetzt.

3. Verfahren nach Ansprüchen 1 und 2, dadurch gekennzeichnet, daß man Katalysatoren einsetzt, die Nebengruppenelemente in elementarer Form oder in Form von Verbindungen enthalten.

4. Verfahren nach Ansprüchen 1 bis 3, dadurch gekennzeichnet, daß man Katalysatoren einsetzt, die Nebengruppenelemente in elementarer Form oder in Form von Verbindungen auf Trägermaterial enthalten.

5. Verfahren nach Ansprüchen 1 bis 4, dadurch gekennzeichnet, daß man pro Mol Edukt 1,8 bis 50 mol Wasserstoff einsetzt.

6. Verfahren nach Ansprüchen 1 bis 5, dadurch gekennzeichnet, daß man mit einem gasförmigen Edukt an einem fest angeordneten Katalysator arbeitet.

7. Verfahren nach Ansprüchen 1 bis 6, dadurch gekennzeichnet, daß man es bei Temperaturen von 50 bis 500°C durchführt.

8. Verfahren nach Ansprüchen 1 bis 7, dadurch gekennzeichnet, daß man es bei Drucken von 0,1 bis 50 bar durchführt.

## Claims

1. Process for preparing 1,1,1,3,3-pentafluoropropane, characterized in that fluorinated and chlorinated propane derivatives of the formula (I)

$$CF_3\text{-}CClX\text{-}CF_2Cl \quad (I)$$

in which

X represents hydrogen or chlorine,

are catalytically hydrogenated.

2. Process according to Claim 1, characterized in that 1,1,1,3,3-pentafluoro-2,3-dichloropropane or 1,1,1,3,3-pentafluoro-2,2,3-trichloropropane or any desired mixtures of the two are used.

3. Process according to Claims 1 and 2, characterized in that the catalysts used contain subgroup elements in elemental form or in the form of compounds.

4. Process according to Claims 1 to 3, characterized in that the catalysts used contain subgroup elements in elemental form or in the form of compounds on support material.

5. Process according to Claims 1 to 4, characterized in that from 1.8 to 50 mol of hydrogen are used per mole of starting material.

6. Process according to Claims 1 to 5, characterized in that a gaseous starting material is used on a fixed catalyst.

7. Process according to Claims 1 to 6, characterized in that the process is carried out at temperatures of from 50 to 500°C.

8. Process according to Claims 1 to 7, characterized in that the process is carried out at pressures of from 0.1 to 50 bar.

**Revendications**

1. Procédé de préparation du 1,1,1,3,3-pentafluoropropane, caractérisé en ce que l'on soumet des dérivés fluorés et chlorés du propane répondant à la formule (I)

$$CF_3\text{-}CClX\text{-}CF_2Cl \qquad (I)$$

dans laquelle

X représente l'hydrogène ou le chlore,

à une hydrogénation catalytique.

2. Procédé selon la revendication 1, caractérisé en ce que l'on met en oeuvre le 1,1,1,3,3-pentafluoro-2,3-dichloropropane ou le 1,1,1,3,3-pentafluoro-2,2,3-trichloropropane ou un mélange quelconque de ces composés.

3. Procédé selon les revendications 1 et 2, caractérisé en ce que l'on utilise des catalyseurs contenant des éléments des sous-groupes de la classification périodique à l'état élémentaire ou à l'état de composés.

4. Procédé selon les revendications 1 à 3, caractérisé en ce que l'on utilise des catalyseurs contenant des éléments des sous-groupes de la classification périodique à l'état élémentaire ou à l'état de composés sur une matière de support.

5. Procédé selon les revendications 1 à 4, caractérisé en ce que l'on utilise de 1,8 à 50 mol d'hydrogène par mole du produit de départ.

6. Procédé selon les revendications 1 à 5, caractérisé en ce que l'on opère sur le produit de départ à l'état gazeux avec un catalyseur en disposition fixe.

7. Procédé selon les revendications 1 à 6, caractérisé en ce que l'on opère à des températures de 50 à 500°C.

8. Procédé selon les revendications 1 à 7, caractérisé en ce que l'on opère sous des pressions de 0,1 à 50 bars.